# EUROPEAN PATENT SPECIFICATION

(11) **EP 0 832 613 B1**
(45) Date of publication and mention of the grant of the patent: **16.01.2002**
(21) Application number: 96830499.8
(22) Date of filing: 30.09.1996
(51) Int. Cl.: A61B 17/60

(54) **Multidirectional mandibular distractor**
Mehrrichtungs-mandibulare Streckvorrichtung
Extenseur mandibulaire multidirectionel

(43) Date of publication of application: 01.04.1998
(73) Proprietor: EUROPEAN COMMUNITY, 2920 Luxembourg (LU)
(72) Inventor: Caboni, Alessandro, 20148 Milano (IT); Crippa, Angelo, 21020 Taino (IT); Paracchini, Luigi, 28053 Castelletto Ticino (IT)
(74) Representative: Cerbaro, Elena, Dr.

(56) References cited:
- WO-A-88/05287
- WO-A-94/00067
- DE-A- 19 503 609
- US-A- 3 961 854

## Description

The present invention relates to a multidirectional mandibular distractor.

In the treatment of bone diseases or traumas, orthopedic mechanical devices are used to stimulate separate parts of the same bone into given mutual positions.

In particular, devices are known for treating traumas or diseases of the mandible. Congenital diseases of the mandible include the Tracher-Collins and Franceschetti syndromes, both of which result in micrognathia, a malformation caused by underdevelopment of the jaw bones, and which is manifested in a noticeable reduction in the chin.

Surgical techniques for treating such diseases consist in cartilage grafting or surgical division of the mandible, often with unsatisfactory results. More recently, a new surgical technique has been devised, which consists in fitting a retractor or distractor to the mandible. In the case of malformation of only one side of the mandible, the distractor is fitted to that side; and, in the case of symmetrical malformation, the distractor is fitted to both sides.

The distractor comprises a central portion; two arms extending from opposite sides of the central portion and connected to it by respective spherical joints; and two slides, each movable axially and positionable discretely along a respective arm. More specifically, to enable discrete positioning of the slides, each presents a connecting element, which meshes with teeth on the respective arm to define a number of axially-fixed mutual positions.

Before fitting the distractor, incisions are made in the bone tissue (in particular, the cortex) along a lateral intermediate portion of the mandible, normally close to the gonion, and along a cross section of the mandible to divide it into two parts joined solely by the bone marrow inside the resected portion of the bone tissue.

The distractor is fitted to the mandible at three points by means of self-threading screws. More specifically, the central portion is fitted close to the surgically resected portion, and the slides to the respective parts separated by the resection, e.g. the upward branch and/or the body of the mandible. Once fitted, the distractor keeps the two parts of the mandible a given distance apart to leave the bone marrow underlying the incision exposed.

Once the distractor is fitted to the mandible, the slides are repositioned along the respective arms, normally several times in the course of treatment, to dislocate the two parts of the mandible.

The slides are repositioned manually by releasing them from one tooth and engaging them inside another along the respective arms, and are locked in the new position by means of a fastening element, e.g. a screw.

In the space of a few weeks, bone is gradually formed about the exposed bone marrow to join the two surgically resected parts.

An example of the above distractor may be found in DE 195 03 609; moreover PCT/DE88/00017 discloses an external bone synthesis aid.

The above device presents several drawbacks. Firstly, as stated, the position of each slide in relation to the respective arm may only be adjusted discretely, i.e. the slide may only assume successive positions separated by a distance equal to the spacing of the teeth along the arm, and the adjustment itself is a complex process requiring a skilled physician.

Secondly, the distractor itself is made of steel, which, in addition to being heavy and insufficiently elastic, also involves problems of biocompatibility.

Finally, known devices are bulky and, in addition to being inevitably unsightly, involve a certain amount of risk in the event of accidental impact by external bodies.

It is an object of the present invention to provide a multidirectional mandibular distractor designed to overcome the aforementioned drawbacks typically associated with known devices.

According to the present invention, there is provided a multidirectional mandibular distractor comprising a central portion which is to be fitted to a mandible; a first and second straight element extending from opposite sides of said central portion; first and second articulated joints interposed respectively between said first and second straight elements and said central portion; a first and second slide movable respectively along said first and second straight elements, and connectable respectively to a first and second part of said mandible; first retaining means interposed between said slides and said straight elements, and for sliding each of said slides along a respective plane; and second retaining means interposed between said slides and said straight elements, and for stably positioning each said slide along a respective straight element; characterized in that said second retaining means are adjustable continuously.

A preferred non-limiting embodiment of the present invention will be described by way of example with reference to the accompanying drawings, in which:
Figure 1 shows a front view of the mandibular distractor according to the teachings of the present invention;
Figure 2 shows a section of the distractor along line II-II in Figure 1;
Figure 3 shows a section of the distractor along line III-III in Figure 1;
Figure 4 shows one example of application of the distractor according to the present invention.

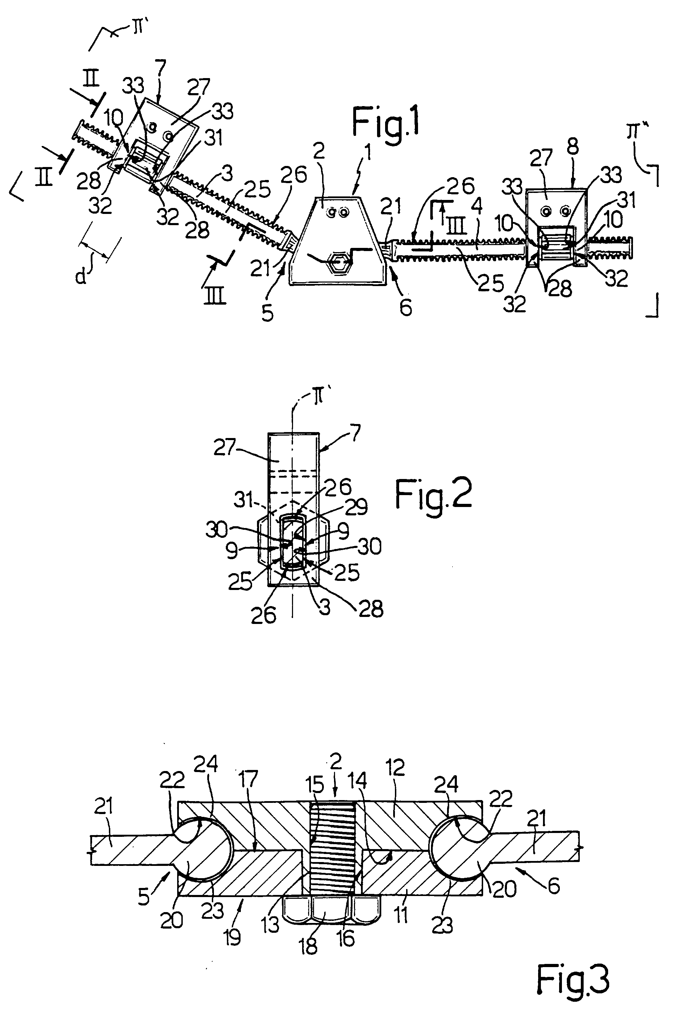

Number 1 in Figure 1 indicates a multidirectional mandibular distractor made entirely of titanium and comprising a substantially flat trapezoidal central portion 2; a first and second straight element 3, 4 extending from opposite sides of central portion 2; first and second articulated joints 5, 6 (shown in detail in Figure 3) interposed respectively between first and second straight elements 3, 4 and central portion 2; and a first and second slide 7, 8 movable respectively along first and second straight elements 3, 4.

Distractor 1 comprises first retaining devices 9 (Figure 2) interposed between slides 7, 8 and respective straight elements 3, 4, and for sliding each slide 7, 8 along a respective plane π', π"; and second retaining devices 10 interposed between slides 7, 8 and respective straight elements 3, 4, and for stably positioning and continuously adjusting each slide 7, 8 along respective straight element 3, 4.

First and second retaining devices 9, 10 are described in more detail later on.

As shown in Figure 3, central portion 2 comprises a first and second flat plate 11, 12 placed one on top of the other and each substantially in the form of an isosceles trapezium.

More specifically, second plate 12 presents an integral cylindrical tubular appendix 13 extending perpendicularly from a face 14 of plate 12, and which is threaded internally (not shown) and defines an inner cylindrical cavity 15 extending the full length of appendix 13 and through plate 12.

Plate 11 presents a circular through hole 16 of a diameter slightly larger than the outside diameter of appendix 13, and which houses appendix 13 when plate 11 is positioned with one face 17 contacting face 14 of plate 12. Plates 11 and 12 are connected stably to each other by a screw 18, which engages cavity 15, and the head of which rests against the opposite face 19 of first plate 11.

Each articulated joint 5, 6 (Figure 3) comprises a spherical element 20 extending integrally from one end 21 of a respective straight element 3, 4; and a spherical seat 22 formed in central portion 2 and housing spherical element 20. More specifically, spherical seat 22 is defined by respective spherical impressions 23, 24 facing each other and formed in respective plates 11, 12.

Each straight element 3, 4 (Figure 2) presents a rectangular cross section with the short sides defining circumferential portions, and with straight long sides corresponding to two flat lateral portions 25 of straight element 3, 4. More specifically, lateral portions 25 are parallel to each other, are located on either side of the axis of straight element 3, 4, and extend the full length of straight element 3, 4; and the circumferential portions of the cross section correspond to threaded portions 26 of straight element 3, 4, also extending the full length of straight element 3, 4.

Each slide 7, 8 is C-shaped and comprises a substantially parallelepiped main portion 27, and two parallel straight arms 28 (Figure 1) extending integrally from the same side of main portion 27, separated by a given distance d, and presenting respective facing holes 29 (one shown in Figure 2). Holes 29 permit the passage of a respective straight element 3, 4, are substantially rectangular, and are defined, among other things, by facing flat walls 30, which contact respective lateral portions 25 to prevent slide 7, 8 from rotating about the axis of straight element 3, 4, and which thus define said first retaining devices 9.

Each of said second retaining devices 10 comprises a sliding element 31 slidable continuously along straight element 3, 4 and interposed between arms 28 of respective slide 7, 8. In the embodiment shown, sliding element 31 is defined by a nut slightly smaller axially than distance d, and presenting opposite end portions 32 contacting respective inner faces 33 of arms 28, so that nut 31 assumes an axially-fixed position in relation to slide 7, 8. Nut 31 is threaded internally to engage with threaded portion 26 of straight element 3, 4.

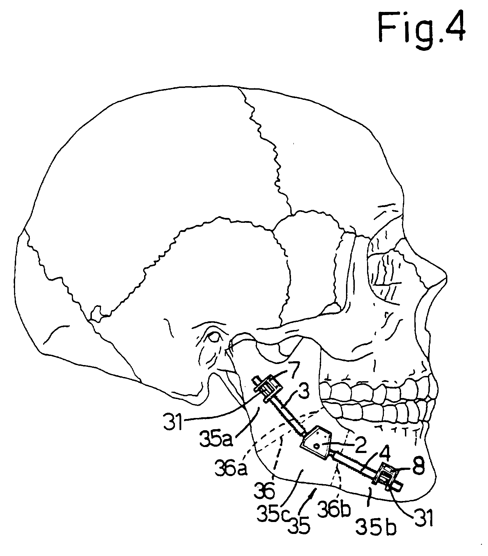

In actual use, distractor 1 is fitted to a mandible 35, as shown in Figure 4. Prior to fitment of distractor 1, one or more incisions are made in the cortex, along a cross section of the mandible, to divide mandible 35 into two or more portions 35a, 35b, 35c joined solely by the bone marrow inside the resected bone tissue portion; central portion 2 is fitted in a lateral portion of the mandible (e.g. at the gonion); and slides 7 and 8 are fitted, e.g. by means of screws (not shown), to respective first and second portions 35a, 35b separated by the incision (e.g. to the upward branch and close to the central portion of the mandible).

Once distractor 1 is fitted, the position of slides 7, 8 in relation to central portion 2 may be adjusted at any time to adjust the pressure applied to and for separating portions 35a, 35b, by simply turning nut 31, which, as it travels in relation to straight element 3, 4, pushes forward respective slide 7, 8, which, as stated, is axially integral with nut 31.

First retaining devices 9 prevent slides 7, 8 from rotating in relation to respective straight elements 3, 4, so that slides 7, 8 are forced to travel along respective planes π', π".

The advantages of the distractor according to the present invention are as follows. Firstly, slides 7, 8 may be positioned continuously and micrometrically along respective straight elements 3, 4 by virtue of the screw-nut screw connection of nuts 31 and straight elements 3, 4. Secondly, adjustment of slides 7, 8 is extremely straightforward and, requiring no complex maneuvering of the slides, may even be performed by the patient himself.

Thirdly, the fact that the distractor is made entirely of titanium provides for greater biocompatibility, thus eliminating the risk of infection, and for a considerable reduction in weight, with obvious advantages for the patient. Moreover, the greater elasticity of titanium as compared with steel enables the distractor to adapt to the bone structure of the mandible as it grows.

Finally, the distractor is of compact design, thus safeguarding against injury as a result of impact by external bodies.

The above advantages provide for a practical, highly effective distractor capable of generating the growth of over 2 cm of bone at the incision.

Clearly, changes may be made to the multidirectional mandibular distractor as described and illustrated herein without, however, departing from the scope of the present invention.

## Claims

1. A multidirectional mandibular distractor comprising a central portion (2) which is to be fitted to a mandible (35); a first and second straight element (3, 4) extending from opposite sides of said central portion (2); first and second articulated joints (5, 6) interposed respectively between said first and second straight elements (3, 4) and said central portion (2); a first and second slide (7, 8) movable respectively along said first and second straight elements (3, 4), and connectable respectively to a first and second part (35a, 35b) of said mandible (35); first retaining means (9) interposed between said slides (7, 8) and said straight elements (3, 4), and for sliding each of said slides (7, 8) along a respective plane (π', π"); and second retaining means (10) interposed between said slides (7, 8) and said straight elements (3, 4), and for stably positioning each said slide (7, 8) along a respective straight element (3, 4);
**characterized in that** said second retaining means (10) are adjustable continuously;
said second retaining means (10) comprise a sliding element (31) slidable continuously along said straight element (3, 4); and stop means (32, 33) interposed between said sliding element (31) and said slide (7, 8);
said sliding element comprises a tubular body (31) presenting an internal thread; each said straight element (3, 4) comprising at least one threaded portion (26) engaging said internal thread.

2. A multidirectional mandibular distractor as claimed in Claim 1, **characterized in that** said slide (7, 8) comprises at least two adjacent portions (28) separated from each other; said sliding element (31) being interposed between said adjacent portions (28).

3. A multidirectional mandibular distractor as claimed in Claim 2, **characterized in that** said slide (7, 8) is C-shaped, and comprises a main portion (27) which is to be fitted to said mandible (35), and two arms (28) extending substantially parallel to each other from said main portion (27); said sliding element (31) being interposed between said two arms (28).

4. A multidirectional mandibular distractor as claimed in one of the foregoing Claims **characterized in that** said stop means comprise opposite end portions (32) of said sliding element (31); and walls (33) of said slide, which cooperate in contacting manner with said opposite end portions (32).

5. A multidirectional mandibular distractor as claimed in any one of the foregoing Claims, **characterized in that** said first retaining means (9) comprise facing stop walls (30) of said slide (7, 8); and substantially flat lateral portions (25) of said straight element (3, 4), which contact said stop walls (30).

6. A multidirectional mandibular distractor as claimed in any one of the foregoing Claims, **characterized in that** said first and second articulated joints comprise first and second spherical articulated joints (5, 6).

7. A multidirectional mandibular distractor as claimed in any one of the foregoing Claims, **characterized in that** said central portion (2) comprises at least a first and second plate (11, 12) placed one on top of the other; and connecting elements (18) interposed between said plates (11, 12).

8. A multidirectional mandibular distractor as claimed in Claim 7 dependent on Claim 6, **characterized in that** each spherical articulated joint (5, 6) comprises aspherical element (20) at one end (21) of said straight element (3, 4); and a spherical seat (22) formed in said central portion (2) and housing said spherical element (20); said spherical seat (22) being defined by respective spherical impressions (23, 24) facing each other and formed respectively in said first and second plates (11, 12).

9. A multidirectional mandibular distractor as claimed in any one of the foregoing Claims, **characterized by** being made of titanium.

## Patentansprüche

1. In mehreren Richtungen wirksames Kiefer-Streck-organ, mit einem Zentralbereich (2), der an einem Kiefer (35) befestigt werden soll, mit zwei geraden Elementen (3, 4), die an entgegengesetzten Seiten aus dem Zentralbereich (2) vorstehen, mit zwei Gelenkverbindungen (5, 6) zwischen je einem der beiden geraden Elemente (3, 4) und dem Zentralbereich (2), mit zwei Gleitgliedern (7, 8), die entlang je eines der beiden geraden Elemente (3, 4) beweglich sind und an je einem Teil (35a, 35b) des Kiefers (35) befestigt werden können, mit ersten Haltemitteln (9) zwischen jedem Gleitglied (7, 8) und dem zugeordneten geraden Element (3, 4), sodaß die Gleitglieder (7, 8) je entlang einer Ebene (π', n") gleiten können, und mit zweiten Haltemitteln (10) zwischen jedem Gleitglied (7, 8) und dem zugeordneten geraden Element (3, 4), sodaß jedes Gleitglied (7, 8) entlang des zugeordneten geraden Elements (3, 4) stabil positioniert werden kann, **dadurch gekennzeichnet, daß** die zweiten Haltemittel (10) kontinuierlich justierbar sind, daß die zweiten Haltemittel (10) ein gleitendes Element (31), das kontinuierlich entlang des geraden Elements (3, 4) gleiten kann, und Anschlagmittel (32, 33) enthält, die zwischen dem gleitenden Element (31) und dem Gleitglied (7, 8) liegen, und daß das gleitende Element (31) einen rohrförmigen Körper mit einem Innengewinde besitzt und jedes gerade Element (3, 4) mindestens einen mit dem Innengewinde zusammenwirkenden Gewindebereich (26) aufweist.

2. In mehreren Richtungen wirksames Kiefer-Streck-organ nach Anspruch 1, **dadurch gekennzeichnet, daß** das Gleitglied (7, 8) mindestens zwei voneinander getrennt angeordnete benachbarte Bereiche (28) aufweist und daß das gleitende Element (31) zwischen diese benachbarten Bereiche (28) eingefügt ist.

3. In mehreren Richtungen wirksames Kiefer-Streck-organ nach Anspruch 2, **dadurch gekennzeichnet, daß** das Gleitglied (7, 8) die Form eines Buchstaben C besitzt und einen Kernbereich (27), der am Kiefer (35) befestigt werden soll, sowie zwei Arme (28) besitzt, die im wesentlichen zueinander parallel von dem Kernbereich (27) ausgehen, wobei das gleitende Element (31) zwischen diese beiden Arme (28) eingesetzt ist.

4. In mehreren Richtungen wirksames Kiefer-Streck-organ nach einem der vorstehenden Ansprüche, **dadurch gekennzeichnet, daß** zu den Anschlagmitteln einander gegenüberliegende Endbereiche (32) des gleitenden Elements (31) sowie Wände (33) des Gleitglieds gehören, die mit den gegenüberliegenden Endbereichen (32) in Kontakt stehen.

5. In mehreren Richtungen wirksames Kiefer-Streck-organ nach einem der vorstehenden Ansprüche, **dadurch gekennzeichnet, daß** zu den ersten Haltemitteln (9) einander gegenüberliegende Anschlagwände (30) des Gleitglieds (7, 8) und im wesentlichen flache Seitenbereiche (25) des geraden Elements (3, 4) gehören, die mit den Anschlagwänden (30) in Kontakt stehen.

6. In mehreren Richtungen wirksames Kiefer-Streck-organ nach einem der vorstehenden Ansprüche, **dadurch gekennzeichnet, daß** die beiden Gelenkverbindungen von ersten und zweiten Kugelgelenken (5, 6) gebildet werden.

7. In mehreren Richtungen wirksames Kiefer-Streck-organ einem der vorstehenden Ansprüche, **dadurch gekennzeichnet, daß** der Zentralbereich (2) zumindest eine erste und eine zweite Platte (11, 12), die aufeinanderliegen, und Verbindungselemente (18) zur Verbindung dieser Platten (11, 12) aufweist.

8. In mehreren Richtungen wirksames Kiefer-Streck-organ nach Anspruch 7 in Abhängigkeit von Anspruch 6, **dadurch gekennzeichnet, daß** jede Gelenkverbindung (5, 6) ein sphärisches Element (20) an einem Ende (21) des geraden Elements (3 bzw. 4) und einen sphärischen Sitz (22) aufweist, der in dem Zentralbereich (2) ausgebildet ist und das sphärische Element (20) aufnimmt, wobei der sphärische Sitz (22) durch sphärische Aushöhlungen (23, 24) definiert wird, die einander gegenüberliegen und in der ersten beziehungsweise zweiten Platte (11, 12) ausgebildet sind.

9. In mehreren Richtungen wirksames Kiefer-Streck-organ nach einem der vorstehenden Ansprüche, **dadurch gekennzeichnet, daß** es aus Titan ist.

## Revendications

1. Extenseur mandibulaire multidirectionnel comportant une partie centrale (2) qui doit être adaptée sur une mandibule (35); un premier et un second élément rectiligne (3, 4) s'étendant à partir de côtés opposés de ladite partie centrale (2); des première et seconde jonctions articulées (5, 6) interposées respectivement entre lesdits premier et second éléments rectilignes (3, 4) et ladite partie centrale (2); un premier et un second coulisseau (7, 8) mobiles respectivement le long desdits premier et second éléments rectilignes (3, 4), et pouvant être connectés respectivement à une première et une seconde partie (35a, 35b) de ladite mandibule (35); des premiers moyens de retenue (9) interposés entre lesdits coulisseaux (7, 8) et lesdits éléments rectilignes (3, 4), et pour faire coulisser chacun desdits coulisseaux (7, 8) le long d'un plan respectif (π', π"); et des seconds moyens de retenue (10) interposés entre lesdits coulisseaux (7, 8) et lesdits éléments rectilignes (3, 4), et pour positionner de manière stable chacun desdits coulisseaux (7, 8) le long d'un élément rectiligne respectif (3, 4);
**caractérisé en ce que** lesdits seconds moyens de retenue (10) sont ajustables de manière continue;
lesdits seconds moyens de retenue (10) comportent un élément coulissant (31) pouvant coulisser de manière continue le long dudit élément rectiligne (3, 4); et des moyens d'arrêt (32, 33) interposés entre ledit élément coulissant (31) et lesdits coulisseaux (7, 8);
ledit élément coulissant comporte un corps tubulaire (31) présentant un filetage intérieur; chacun desdits éléments rectilignes (3, 4) comportant au moins une partie filetée (22) en prise avec ledit filetage intérieur.

2. Extenseur mandibulaire multidirectionnel selon la revendication 1, **caractérisé en ce que** ledit coulisseau (7, 8) comporte au moins deux parties adjacentes (28) séparées l'une de l'autre; ledit élément coulissant (31) étant interposé entre lesdites parties adjacentes (28).

3. Extenseur mandibulaire multidirectionnel selon la revendication 2, **caractérisé en ce que** ledit coulisseau (7, 8) est en forme de C, et comporte une partie principale (27) qui doit être adaptée sur ladite mandibule (35), et deux bras (28) s'étendant sensiblement parallèlement l'un à l'autre à partir de ladite partie principale (27); ledit élément coulissant (31) étant interposé entre lesdits deux bras (28).

4. Extenseur mandibulaire multidirectionnel selon l'une des revendications précédentes, **caractérisé en ce que** lesdits moyens d'arrêt comportent des parties d'extrémité opposées (32) dudit élément coulissant (31); et des parois (33) dudit coulisseau, qui coopèrent par contact avec lesdites parties d'extrémité opposées (32).

5. Extenseur mandibulaire multidirectionnel selon l'une quelconque des revendications précédentes, **caractérisé en ce que** lesdits premiers moyens de retenue (9) comportent des parois d'arrêt en vis-à-vis (30) desdits coulisseaux (7, 8); et des parties latérales sensiblement plates (25) desdits éléments rectilignes (3, 4) qui sont en contact avec lesdites parois d'arrêt (30).

6. Extenseur mandibulaire multidirectionnel selon l'une quelconque des revendications précédentes, **caractérisé en ce que** lesdites première et seconde jonctions articulées comportent des première et seconde jonctions articulées sphériques (5, 6).

7. Extenseur mandibulaire multidirectionnel selon l'une quelconque des revendications précédentes, **caractérisé en ce que** ladite partie centrale (2) comporte au moins une première et une seconde plaque (11, 12) placées l'une au-dessus de l'autre; et des éléments de connexion (18) interposés entre lesdites plaques (11, 12).

8. Extenseur mandibulaire multidirectionnel selon la revendication 7 dépendante de la revendication 6, **caractérisé en ce que** chaque jonction articulée sphérique (5, 6) comporte un élément sphérique (20) à une première extrémité (21) dudit élément rectiligne (3, 4); et un siège sphérique (22) formé dans ladite partie centrale (2) et recevant ledit élément sphérique (20); ledit siège sphérique (22) étant défini par des empreintes sphériques respectives (23, 24) dirigées l'une vers l'autre et formées respectivement dans lesdites première et seconde plaques (11, 12).

9. Extenseur mandibulaire multidirectionnel selon l'une quelconque des revendications précédentes, **caractérisé en ce qu'**il est fait de titane.
